# EUROPEAN PATENT SPECIFICATION

(11) **EP 3 815 627 B1**
(45) Date of publication and mention of the grant of the patent: **16.03.2022**
(21) Application number: 19020608.6
(22) Date of filing: 31.10.2019
(51) Int. Cl.: A61B 17/12

(54) **DEVICE FOR ADAPTING AN LAA OCCLUDER AFTER IMPLANTATION**
VORRICHTUNG ZUR ANPASSUNG EINES LAA-OKKLUDERS NACH DER IMPLANTATION
DISPOSITIF D'ADAPTATION D'UN DISPOSITIF D'OCCLUSION DE L'APPENDICE AURICULAIRE GAUCHE APRÈS IMPLANTATION

(43) Date of publication of application: 05.05.2021
(73) Proprietor: Peter Osypka Stiftung, 79639 Grenzach-Wyhlen (DE)
(72) Inventor: Osypka, Peter, 79639 Grenzach-Wyhlen (DE)

(56) References cited:
- EP-A1- 3 459 469
- US-A1- 2004 215 230
- US-A1- 2009 306 706

## Description

This invention relates to a device for adapting a LAA occluder after implantation, especially an adjustable custom made occluder for placement within the left atrial appendage (LAA). Left atrial appendage closure (LAAC) is a known treatment strategy for reducing the risk of left atrial appendage blood clots entering the atrial bloodstream and causing a stroke in patients with atrial fibrillation (AF). In case of atrial fibrillation, stroke-causing clots are formed in the left atrium and the left atrial appendage (LAA).

Examples of LAA occluder clinically used for stroke prophylaxis in AF patients are: the Watchman occluder, the Amplatzer occluder and the Lifetech LAmbre occluder.

The Amplatzer occluder is made from a self-expanding nitinol wire mesh comprising a disc and a cylindrical lobe. The occluder named "Amplatzer Cardiac Plug" is available in different sizes having a lobe diameter in the range of 16-30mm and a cover part in the range of 20-36mm.

The Watchman occluder is a parachute shaped self-expanding device having a cylindrical shape. The Watchman occluder is made from a nitinol wire mesh covered with a polyethylene terephthalate cap. The occluder is available in different sizes 21-33mm diameter.

The LAmbre occluder is a nitinol-based, self-expanding device comprising
a hook-embedded umbrella and a cover connected with a short central waist. The occluder is available in different sizes having an umbrella diameter in the range of 16-26mm and a cover diameter in the range of 22-38mm.

US 2004/0215230A1 describes an adjustable LAA comprising a disc connected to a lobe, the lobe comprises at least one restriction element running inside the lobe to control the diameter of the lobe. The restriction element is a flexible line having slip knots.

US2009/0306706A1 describes an ASD occlusion element having a double walled occlusion disk. The occlusion element is radially compressed in the middle region.

EP3459469A1 describes an LAA occluder being longitudinally adjustable by means of a locking wire. The occlusion element is radially compressed in the middle region.

The LAA morphology is complex. Different types are differentiated, for example the "Windsock Type", the "Chicken Wing Type", the "Cauliflower Type" and the "Cactus Type".

The LAA lobe may further be cone shaped or have a double lobe. The orifice of the LAA to the left atrium is called the LAA ostium. The LAA ostium is also irregularly shaped, having a circular, elliptical or elongated shape with a more or less wavy margin.

Due to the extremely complex and heterogeneous morphology of the LAA and the irregular wavy shape of the LAA ostium border, the above mentioned standard occluder may not fulfill the requirement of complete ostial seal for patients having an irregularly shaped or a very elliptical shaped LAA ostium. After the occluder is implanted there may remain a small opening (leakage) through which blood clots can enter into the left atrium. Leaks were graded as minor (< 1mm), moderate (1-3mm) or major (>3mm). Device related thrombus is noted at about 3-5% of the patients after implantation of the occluder.

### (see European Society of Cardiology ESC , doi:10.1093/europace/euz258)

There is still a need to provide a LAA occluder allowing complete seal and taking into account said complex morphology which is different for every patient.

Thus, the aim of the present invention is to provide a LAA occluder configured to be inserted into a patient's left atrial appendage cavity. The shape and size of the occluder should be adjustable to the shape and size of the patient's LAA cavity after the occluder has been inserted and even after the occluder has been fixed and while the occluder remains inserted.

The invention thus relates to a medical occluder made of self-expanding shape memory material configured to be inserted into a patient's left atrial appendage cavity, said occluder comprising a disc connected to a lobe, the lobe is configured to move between a first size-restricted configuration and at least one second enlarged configuration, the restricted configuration is 1-40% smaller ib diameter than the enlarged configuration, the lobe comprises at least one restriction element running inside the lobe to control the diameter of the lobe.

The first size-restricted configuration is a partly expanded configuration. This size restricted configuration is the configuration of the occluder after being fully released out of the delivery catheter. The second enlarged configuration is the fully expanded configuration given by the shape memory effect.

The diameter of the LAA occluder in the restricted configuration is 1-40% smaller than the diameter of the LAA occluder in the enlarged configuration, e.g. 5%; 10%; 15%; 20%; 25%, 30% 40%, preferably 5% to 30% or 5% to 20%.

The inventive idea behind the new LAA occluder is that of forming a LAA occluder of shape memory material having a fully expanded configuration and a partly expanded configuration. The fully expanded configuration is given by the shape memory effect of the occluder material. The partly expanded configuration is provided by using an expansion restriction element which runs inside the lobe of the LAA occluder. The expansion restriction element is fixed on the lobe and is able to block the expansion of the LAA occluder given by the shape memory effect. If required the restricting effect is easily reversible just by separating parts of the restriction element or by cutting through the restriction element. Thereby the tension of the restriction element decreases allowing expansion of the implanted occluder.

The inventive occluder comprises a lobe and a disc. The lobe is placed into the LAA cavity and fixed in the upper region called landing zone.

The disc seals the ostium. The disc may have various shapes such as for example circular elliptical, pear shaped and the like. Preferably the disc is custom made meaning that the disc is shaped according to exact patient data such as ultrasonic data, transoesophagal echocardiography (TOE) or cardiac computer tomography angiography (CCTA) or other known imaging techniques. Those data are collected before implantation of the occluder.

The lobe may also have various shapes. It is common knowledge that the shape memory material can be formed and tempered to give many different shapes for example cylindrical, spherical, ellipsoidal, balloon shaped and the like. The lobe may have a smooth surface or may have protrusions. The lobe may be symmetrically or asymmetrically shaped. Asymmetric forms are e.g. those forms with decreasing or increasing diameter from distal to proximal. The lobe may be straight or bent. Preferably the lobe is also custom made meaning that the lobe is shaped according to patient data as mentioned above but the diameter is slightly larger than the exact diameter of patient's LAA, as will be explained in more detail below.

A standard size is for example a cylindrical lobe having a length of 10-40mm and a diameter of 10 to 40mm.

The custom made occluder may be manufactured using 3-D computer graphics as described in WO2016087145.

The method comprises the following steps:
a) generating a three-dimensional computer graphic of the patient's left atrial appendage (LAA) based on medical images of the LAA,
b) transferring the data of the computer graphic to a 3-D printer device,
c) forming a LAA model by 3-D printing, said model being an exact reconstruction of the patient's LAA,
d) manufacturing the occluder comprising a disc part and a lobe part one following the other in a proximal-distal direction according to the LAA model obtained in step c) to obtain an occluder conforming in size and shape to the patient's LAA.

The occluder (lobe and disc) may be shaped of any biocompatible self expanding material. Preferably the occluder is shaped of a shape memory material.

Shape memory material is a material which recovers from a deformed shape to a pre-formed shape. The shape memory material may be a shape memory alloy, a shape memory steel alloy or shape memory polymer. Shape memory materials are known. The most preferred shape memory metal is nitinol. In one embodiment the occluder is thus formed of nitinol.

Representative shape memory polymers are e.g. polyurethanes, polyethylene terephthalate (PET), polyethylene oxides (PEO) or block copolymers containing a silicone segment. The shape memory polymer may be a carrier for magnetic nanoparticles.

The occluder has an expandable mesh structure or braid structure. These structures are well known.

The occluder is preferably braided, preferably braided of nitinol wires and may be shaped using various braid patterns. The braid patterns may differ in the number of wires and the wire thickness. Thus the braid may be shaped having different densities. The braid density may be uniform or may vary in part.

The nitinol braid comprises a multiplicity of wire elements, preferably at least 30 wire elements, more preferably 30 to 100 wire elements having a diameter of about 0.03 to 0.2 mm. Wire elements may be wires, filaments, threads or the like.

The stiffness of the braid depends on the wire diameter and the braid pattern. Twin wires can also be used. Thus various stiff braids can be provided.

The ends of the wires of the braid are brought together and held by a closure assembly, said closure assembly forming the distal end. Any closure assembly capable of preventing the wires from escaping may be used. Non-limiting examples are a sleeve or a ring to which the ends are welded or an assembly in which the ends are welded together to form a welded ball. The ends are preferably laser welded. The welded end has preferably a slot for engaging the delivery catheter

The braid may further comprise at least one layer of occluding material in the form of a coating or a patch. The coating or the patch improves the sealing performance.

The coating may be on the outside and/or on the inside of the braiding. The coating may cover the disc or the whole occluder (disc and lobe).The coating is suitably made of silicone, polyurethane, polyester such as PET or polytetrafluoroethylene (PTFE). In one embodiment the coating is silicone. The silicone coating may function as drug carrier. The drug carrier allows the release of any drug, e.g. blood thinners.

The occluder may be equipped with sensors or electrodes for transmitting signals.

The disc and lobe are braided in one piece or disc and lobe are connected using for example a screw-in thread. Such an arrangement has the advantage that the disc is replaceable or repositionable.

The disc may be adjusted using a catheter having tentacles, using a so called catcher. The catcher may be used for turning the disc of the occluder helping to reposition the occluder. For repositioning reasons a thread or a double thread may be attached to the proximal end of the disc. The thread leads to the proximal end of the handle of the delivery catheter. The thread can be easily removed just by pulling.

As mentioned above the aim of the present invention is the fact that the lobe has two configurations, a fully expanded configuration and a partly expanded configuration.

In the fully expanded configuration the lobe of the occluder has a diameter which is larger than the diameter of patient's LAA cavity in the so called landing zone (the region where the lobe of the occluder is fixed). The diameter of the landing zone is determined according to exact patient data such as ultrasonic data, transesophageal echocardiography (TOE) or cardiac computer tomography angiography (CCTA) or other known imaging techniques. Those data are collected before implantation of the occluder. The diameter is larger means 1-40%, e.g. 5%; 10%; 15%; 20%; 25%, 30% 40% larger than the diameter of the landing zone, preferably 5% to 30% or 5% to 20%.

Due to the diameter of the lobe being larger than the diameter of patient's LAA the lobe presses against the tissue of the LAA cavity. The tissue now firmly surrounds the lobe guaranteeing perfect seal.

In the partly expanded configuration the lobe of the occluder has the diameter of patient's LAA in the landing zone. The occluder should fit, however due to the soft tissue of LAA cavity or it's morphology the sealing effect of the partly expanded occluder may not be optimal. The shape and size of the partly expanded occluder is given by the expansion restriction element.

The expansion restriction element may be any element which is able to cause a reduction in diameter of the fully expanded occluder or to block fully expansion. The element may be a biocompatible thread, a screw-in thread, a coil, a helical element and the like.

The expansion restriction element is at least one thread such as a surgical thread running inside the lobe of the occluder. Said at least one thread is tightening together or narrowing the fully expanded configuration. The number of threads and their position can vary. As an example 1, 2 or 3 threads are present running in transverse direction at distal, proximal and central parts of the lobe. The thread may be a single thread or a pair of threads.

In one embodiment two or three threads run in parallel inside the lobe. The threads have different length. So the fully expanded configuration can be reached stepwise by successively cutting the threads one after the other.

According to the present invention, at least a pair of threads crossing each other is present. The pair of threads runs inside the lobe of the occluder in transverse direction and tightens the fully expanded configuration evenly together. Using a pair of threads crossing each other has the advantage of getting an even shape. One pair of threads may be present or two pairs or three pairs or even more pairs.

The shape of the partly expanded occluder can be adjusted by the inventive concept of loosening the expansion restriction element. In case of threads used as expansion restriction element the threads are cut. The cut thread remains inside the lobe. It is not necessary to remove the thread. Loosening the expansion restriction element results in expansion of the lobe due to the shape memory effect. This procedure is a fine adjustment resulting in variable levels of expansion of the partly expanded occluder. The widest possible shape and size of the occluder is the fully expanded configuration. Depending on the number of threads and on the thread guiding individual sections of the occluder can remain less expanded than the expansion due to the shape memory effect. Depending on the number of cut threads protrusions may be formed.

Implantation of the occluder starts by using a delivery catheter and guiding the device in its compressed partly expanded configuration into the LAA cavity. The occluder is released and only partly expands due to the expansion restriction element (e.g. threads) running inside the occluder.

If during or after implantation of the device the result is that in whole or in part a slightly bigger size of the occluder is required, said bigger size can be reached just by cutting at least one of the threads by means of a special cutting stylet being equipped with a cutter. After having cut one thread the lobe has an elliptical shape. A further size enlargement and thus a second size of the occluder can be reached by cutting further threads. Cutting two threads which cross each other results in a circular enlargement. The size adjustment is without implant removal.

It is even possible to selectively enlarge individual sections of the occluder depending on the number of threads and the thread guiding.

By connecting parts of the lobe with threads in longitudinal direction specific parts may also be selectively enlarged.

The threads mentioned above may be nitinol wires as used for the braid, a surgical non-resorbable thread, a polymer thread (polyamide, polyester, polytetrafluorethylene, silicone and the like). Any known non-resorbable surgical suture material can be used. Double threads can also be used. Silicone rings or silicone films can also be used as restriction element.

In one configuration, which does not form part of the present invention, the expansion restriction element is a screw-in thread. The screw-in thread may be made of any biocompatible material such as a shape memory material, e.g. nitinol, an alloy such as MP35N, stainless steel. The screw-in thread may be made of Nitinol and cut out of a tube-like piece of Nitinol. The screw-in thread may also be made by coiling a wire, e.g. a nitinol wire. The screw-in thread runs in a longitudinal direction between the disc and the lobe. The screw-in thread is made of two parts which are screwed within each other. The shape of the occluder is adjusted by turning the screw-in thread. The length of the screw-in thread is thus reduced and the lobe expands in diameter. The advantage of said configuration is that now an efficient method is given of pulling the disc of the occluder into the LAA cavity resulting in a better seal. Thus a double adjustment is given. The position and fit of the disc can be adjusted together with the fit of the occluder lobe.

A further advantage is that the disc can be removed by turning the screw-in thread in the counter direction so that the two parts of the screw-in thread separate. The disc can thus be replaced and further adapted to a more customized model.

After being inserted and placed correctly the occluder is fixed using anchor members. Known anchor techniques for fixing a LAA occluder can be used. Preferably the anchor is tailored to the lobe.

If the lobe is a braid of metal wires the anchor is tailored to the braid comprising at least one wire element or tape element preferably made of the same material as the braid. The tape element may be laser cut. The anchor may be hook shaped at its distal end. Hook shaped means said element bends toward the inner wall of the LAA cavity after the closure device has been inserted. Any kinds of hooks are conceivable. Other kinds of known fixing members such as loops, T-bars or coils of the screw in type may also be used. The length of the anchor depends on the length and morphology of the LAA cavity.

In a configuration, which does not form part of the present invention, a fixing thread extends from the distal end of the lobe to the central part of the lobe. The fixing thread is fixed at the occluder lobe. Such a fixing ensures save delivery and the possibility to reposition the occluder.

The thread used as restriction element may in addition function as anchor element. The thread is fixed to the lobe. The fixing point are small protrusions that can function as anchor.

The occluder is configured to receive a delivery device there through.

A suitable delivery catheter may be the Oscor Destino^{™} having a bi-directional steerable guiding sheath for the introduction and placement of the LAA occluder. The Destino^{™} is available in a size range of 8.5 F, 10 F, and 12 F with true bi-directional tip deflections of 180°.

The cutting stylet is distally flattened and bent.

An advantage of the present invention is that now an efficient method is given to adjust the fit of the LAA occluder after being positioned and possibly fixed. Removal and repositioning is not necessary. Repositioning an occluder especially an occluder having anchor members may result in tissue damage. Thus one aspect of the present invention is to avoid tissue damage.

Illustrative embodiments of the invention and certain configurations, which do not form part of the invention, are described in more detail below with reference to the drawings. The numbering of the drawings is as follows:
- Lobe of the occluder: 1
- disc of the occluder: 2
- welded distal end of the lobe: 3
- Threads: 4, 5, 6, 7
- Helical spring: 8, 9
- LAA cavity: 10
- Landing zone: 11
- Ostium: 12
- Septum: 13
- Atrium: 14
- Leak: 15
- Fixing thread: 16
- Anchor member: 17
- Delivery catheter: 20
- Pusher: 21
- Pulling thread: 22
- Pulling thread: 23
- Fitting: 24, 25
- Cutter: 26
- Stylet: 27
- LAA model: 28
- Expansions range: 30, 31, 32

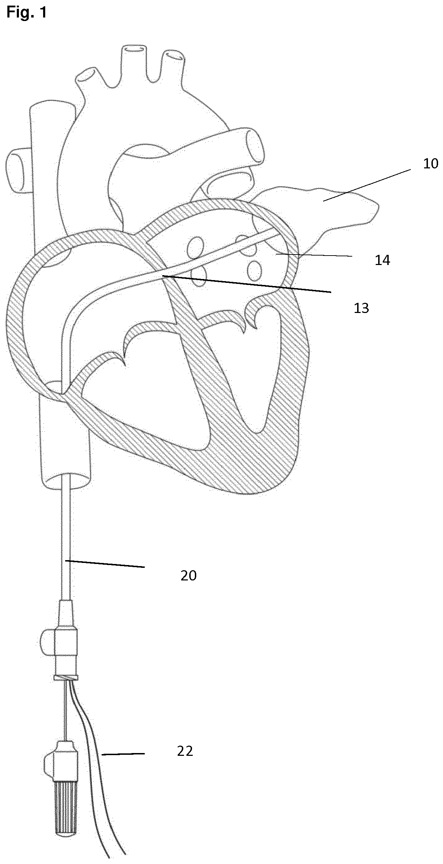

**Fig. 1** Fig. 1 is an overview drawing showing a patient's heart with the left atrial appendage (LAA) (10) and the transseptal access to the LAA via the septum (13) and the left atrium (14). A delivery catheter (20) is guided to the LAA. Pulling threads (22) may be present.

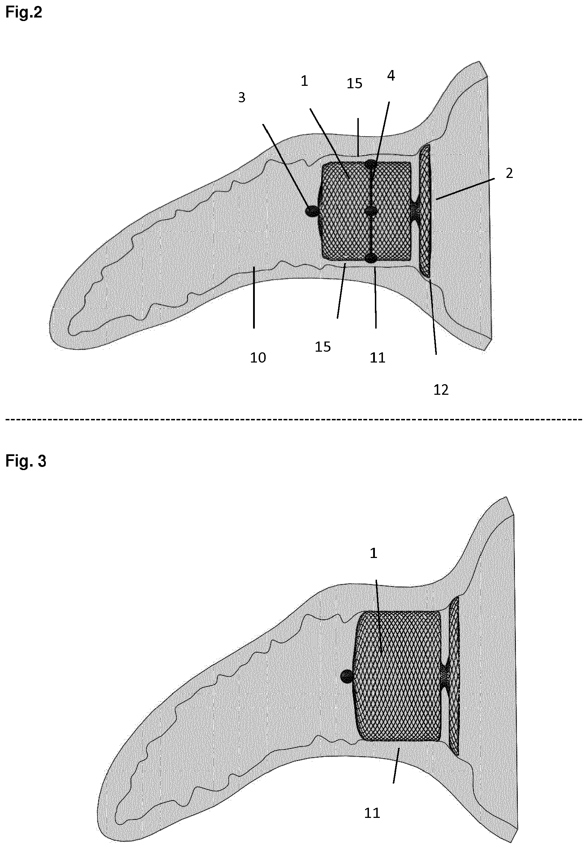

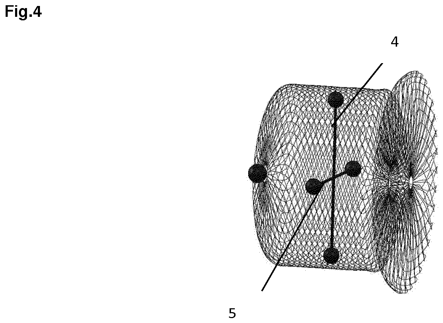

**Fig. 2** is a side view showing the occluder braided of nitinol wires and being inserted into the LAA cavity (10). The occluder comprises a lobe (1) inserted in the so called LAA landing zone (11) and a disc (2) being placed in the ostium (12). The lobe is formed in the partly expanded configuration. The ends of the wires of the braid are laser welded indicated by dot (3). The expansion restriction element is a pair of threads crossing each other. Thread (4) is fixed at opposite parts of the braid (indicated by dots).The dot in the middle indicates the crossing point of the threads (to be seen in detail in Fig. 4. The pair of thread narrows the lobe and blocks the lobe of the occluder to be fully expanded.

Implantation of the occluder starts by using a delivery catheter and guiding the occluder in its compressed partly expanded configuration into the LAA cavity. This first partly expanded configuration corresponds to the patient's LAA the data of which are collected using the above mentioned known imaging techniques. The occluder is released and only partly expands due to the threads running inside the occluder. Fig. 2 illustrates the case that the inserted occluder does not completely seals the landing zone of the lobe. There are small leaks (15) at both sides of the lobe.

**Fig. 3** is a side view showing the LAA occluder of Fig. 2 in the fully expanded configuration. The threads of the occluder shown in Fig. 2 have been cut. Thus, the narrowing effect has been removed and the occluder is expanded to the shape created by the shape memory effect of the tempered nitinol wires. The lobe now presses against the tissue so that the tissue firmly surrounds the lobe. The lobe (1) now completely seals the so called LAA landing zone (11).

**Fig. 4** is a three dimensional view showing the occluder braided of nitinol wires in the partly expanded configuration. Thread (4) and thread (5) cross each other. Thread (4) and thread (5) are each fixed at opposite parts of the braid (indicated by dots).

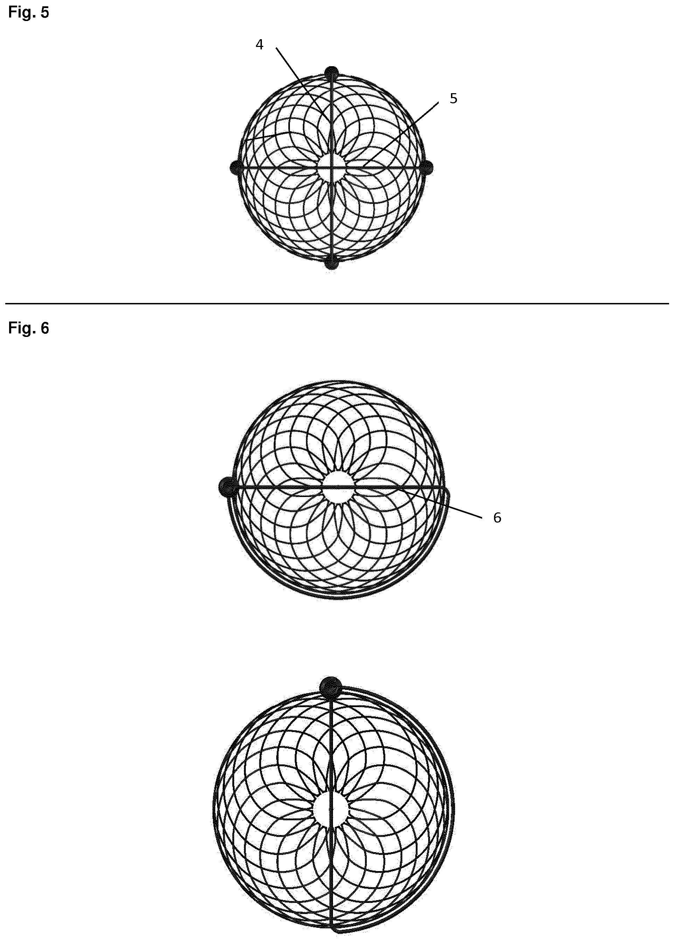

**Fig. 5** is a top view showing that thread (4) and thread (5) cross each other. Thread (4) and thread (5) run inside the occluder and are fixed (knotted) at opposite parts of the braid. (indicated by dots)

**Fig. 6** is a top view showing a further option of arranging the thread. One single thread (6) fixed (knotted) at the lobe (see black dot) runs inside the lobe, is then passed to the surface to run along the surface of the occluder back to the common knot.

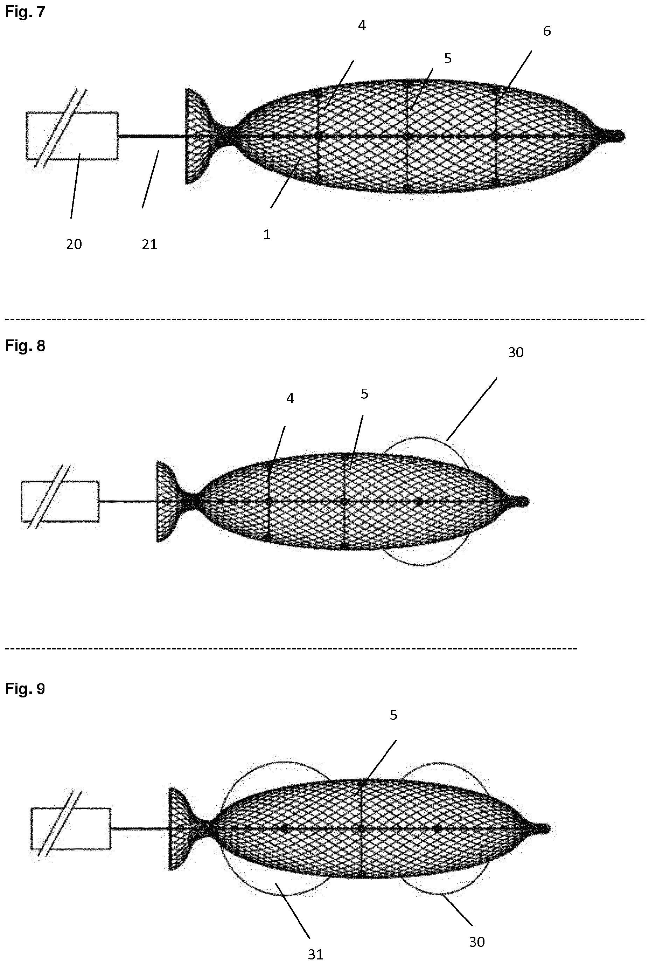

**Fig. 7** is a side view showing an elongated shaped occluder in a partly expanded configuration. The delivery catheter (20) is schematically indicated. A pusher (21) is guided through the occluder in the longitudinal direction. The pusher is necessary to push the occluder out of the delivery catheter. Three pair of threads (4, 5, 6 ) fixed at opposite parts of the braid (indicated by dots) and crossing each other in the middle (indicated by a dot in the middle) are shown. The threads restrict the expansion of the occluder lobe (1) so that the lobe remains in a partly expanded configuration until the threads are cut.

**Fig. 8** is a side view showing the occluder of Fig. 7. One thread of the distally placed pair of threads is cut. Said distal part of the lobe now expands ellipsoidal due to the shape memory effect of the nitinol wire. This expansion is indicated by circle (30). For an even , circular expansion the second thread has to be cut.

**Fig. 9** is a side view showing the occluder of Fig. 8. One thread of the proximally placed pair of threads is cut. Circle (31) indicates that the proximal part of the lobe expands.

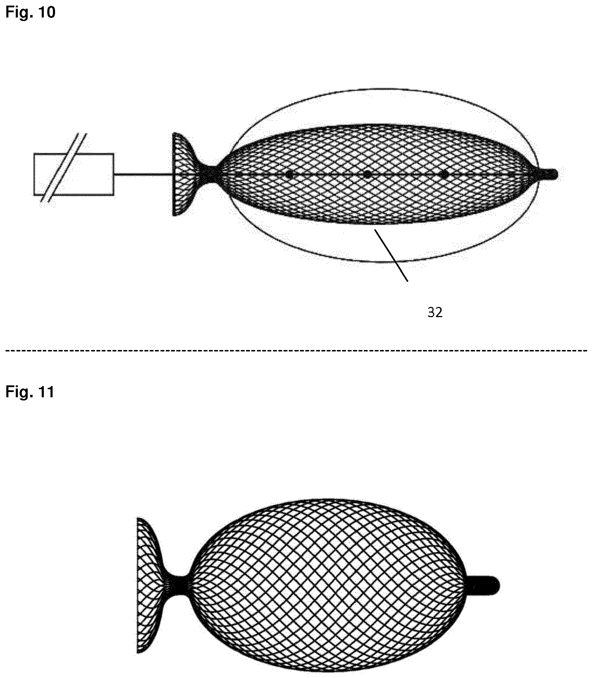

**Fig. 10** shows the occluder of Fig. 9. One thread of the pair of threads placed in the middle has been cut. Circle (32) in the figure indicates the expansion.

**Fig. 11** shows the fully expanded occluder. All the threads are cut.

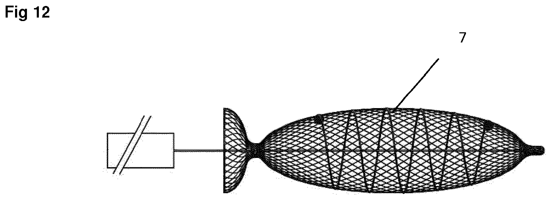

**Fig. 12** shows an elongated shaped occluder having an expansion restriction element in form of a thread (7) extending inside of the lobe and running between the fixing knots (indicated by distal and proximal dot). Thread (7) is helically drawn inside the lobe.

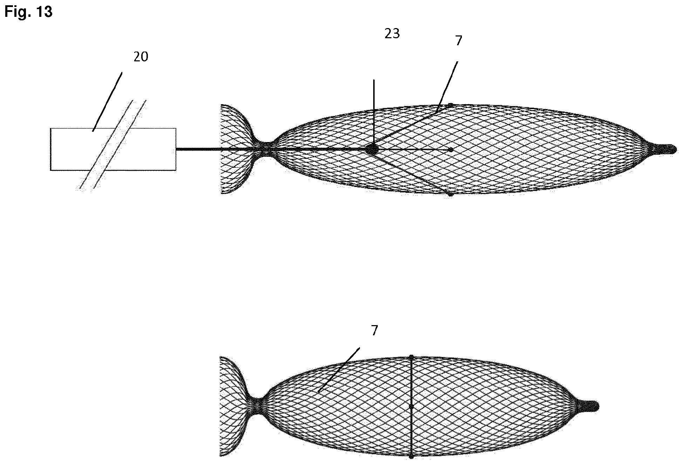

**Fig. 13** is a side view showing an expansions restriction element which is a combination of a thread (7) and a pulling thread (23). The pulling thread is drawn around the thread (7). The upper drawing shows the restricted version. Detaching the pulling thread causes an expansion. This is shown in the drawing below. By cutting thread (7) a further expansion is possible. The expansion can thus be done stepwise.

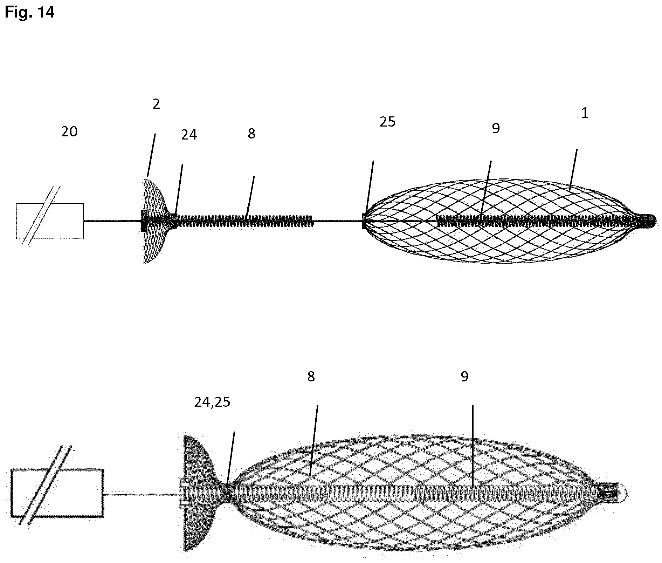

**Fig. 14** shows an occluder having an expansion restriction element in form of a screw-in thread. The two parts of the screw-in thread (8), (9) run in a longitudinal direction between disc (2) and lobe (1). Fittings (24) and (25) attach the lobe (1) to the disc (2). The delivery catheter (20) is schematically indicated. The shape of the occluder is adjusted by turning part (8) and (9) within each other. This is shown in the lower figure. Lobe 1 expands. The disc (2) of the occluder according to Fig. 14 can be removed by turning the screw in thread in the counter direction so that the two parts of screw-in-thread (8) and (9) separate. The two parts run in the opposite direction. The disc can thus be replaced.

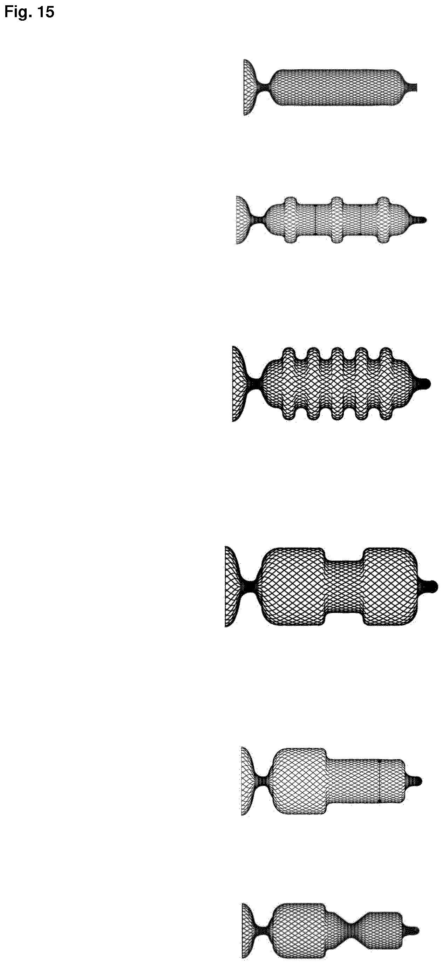

**Fig. 15** gives an overview of various shapes of the lobe.

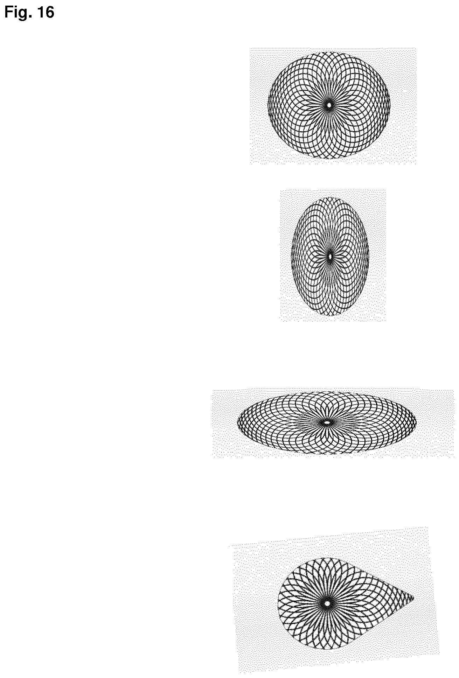

**Fig. 16** shows various shapes of the custom made disc.

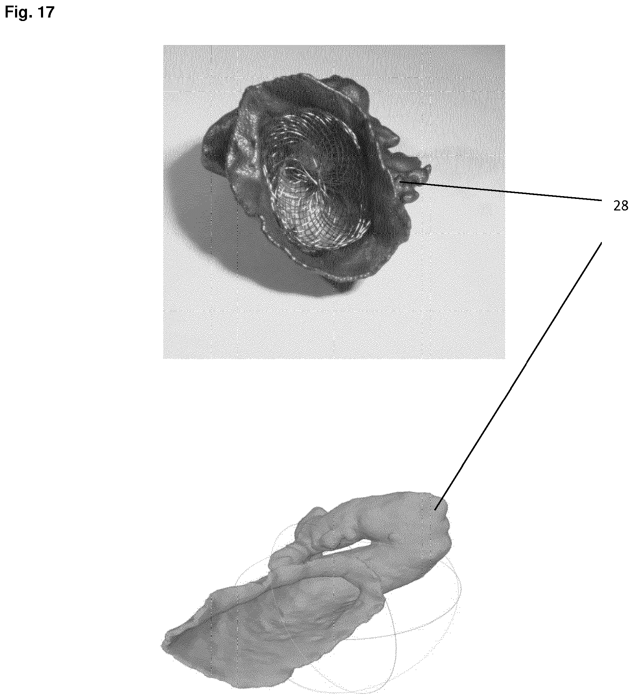

**Fig. 17** illustrates the forming of the custom made occluder using a LAA model (28).

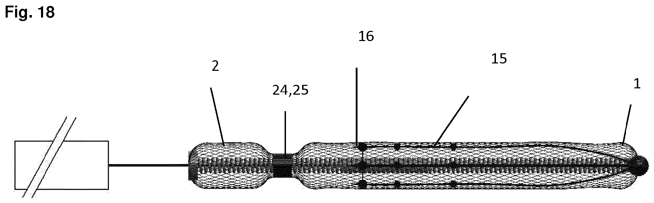

**Fig 18** shows an example of a fixing thread (15) extending from the distal end of the lobe.

The occluder is compressed. Lobe (1) and disc (2) are compressed. Fittings (24) and (25) attach the lobe (1) to the disc (2). The fixing thread is fixed at the occluder lobe (shown by black dots. Such a fixing ensures save delivery and the possibility to reposition the LAA within the landing zone.The open end of the fixing thread (16) functions as anchor member

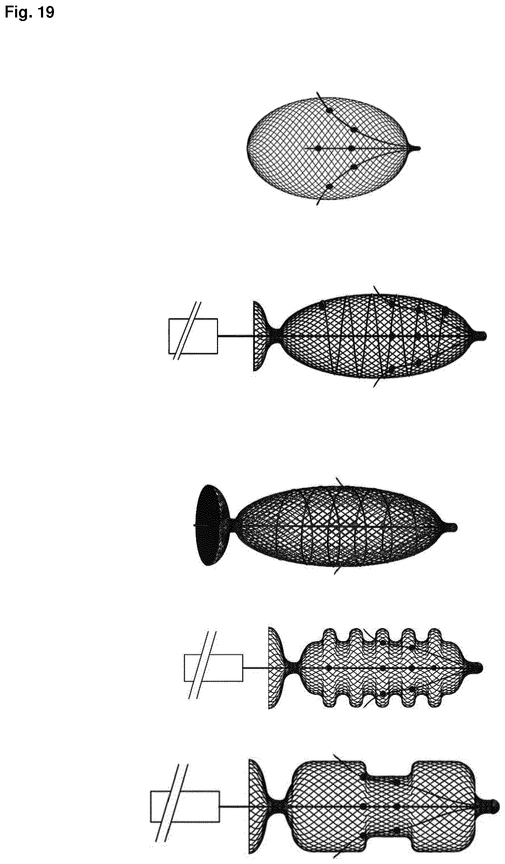

**Fig. 19** shows the occluder having various shaped and being partly expanded. The anchor member is now ready to reach the tissue of the LAA cavity.

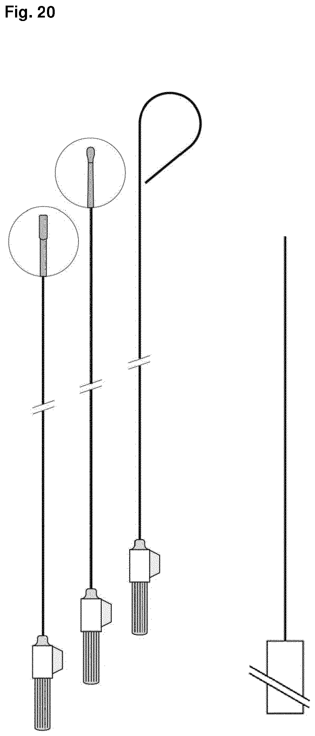

**Fig. 20** shows various pusher.

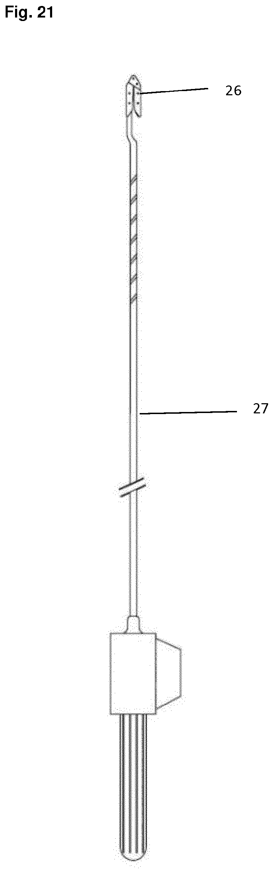

**Fig 21** shows the stylet (27) with cutter (26)

## Claims

1. A medical occluder made of self-expanding shape memory material configured to be inserted into a patient's left atrial appendage cavity, said occluder comprising a lobe (1) and a disc (2), the disc (2) being connected to the lobe (1), the lobe (1) is configured to move between a first size-restricted configuration and at least one second enlarged configuration, the restricted configuration is 1-40% smaller in diameter than the enlarged configuration, the lobe (1) comprises at least one restriction element running inside the lobe to control the diameter of the lobe, **characterised in that** the at least one restriction element is one pair of surgical thread (4,5) running inside the lobe in the transverse direction and crossing each other in the middle of the lobe, each thread (4,5) is fixed at opposite parts of the lobe (1).

2. The occluder according to claim 1 wherein the diameter of the occluder in the size-restricted configuration is 5% to 30% or 5% to 20% smaller than the diameter of the occluder in the enlarged configuration.

3. The occluder according to claim 1 wherein the occluder is a nitinol braid.

4. The occluder according to claim 1 wherein the disc (2) is circular, elliptical or pear shaped.

5. The occluder according to claim 1 further comprising at least one anchor member.

6. The occluder according to claim 1 further comprising a stylet (27) with a cutter (26)

## Patentansprüche

1. Medizinischer Occluder aus selbstexpandierendem Formgedächtnismaterial, der sich zum Einführen in die linke Herzohrhöhle eines Patienten eignet, wobei der Occluder aus einem Verankerungskörper (lobe) (1) und einer Scheibe (2) besteht, wobei die Scheibe (2) mit dem Verankerungskörper (1) verbunden ist; der Verankerungskörper ist so ausgestaltet, dass er zwischen einer ersten verkleinerten Konfiguration und mindestens einer zweiten vergrößerten Konfiguration wechseln kann, wobei der Durchmesser der verkleinerten Konfiguration um 1-40 % kleiner ist als der Durchmesser der vergrößerten Konfiguration, der Verankerungskörper (1) weist mindestens ein Steuerungselement auf, welches innerhalb des Körpers sich befindet und dazu dient den Durchmesser des Verankerungskörpers zu regulieren; **dadurch gekennzeichnet, dass** das Steuerungselement aus zwei chirurgischen Fäden (4,5) besteht, die innerhalb des Verankerungskörpers in Quer-und Längsrichtung verlaufen und sich in der Mitte des Verankerungskörpers kreuzen, wobei jeder Faden (4,5) an sich gegenüberliegenden Enden fixiert ist.

2. Occluder nach Anspruch 1, wobei der Durchmesser des Occluders in der verkleinerten Konfiguration 5 % bis 30 % oder 5 % bis 20 % kleiner ist als der Durchmesser des Occluders in der vergrößerten Konfiguration.

3. Occluder nach Anspruch 1, wobei der Occluder ein Nitinolgeflecht ist.

4. Occluder nach Anspruch 1, wobei die Scheibe (2) kreisförmig, elliptisch oder birnenförmig ist.

5. Occluder nach Anspruch 1, der ferner mindestens ein Ankerelement aufweist.

6. Occluder nach Anspruch 1, der ferner ein Stylet (27) mit einer Schneidvorrichtung (26) aufweist.

## Revendications

1. Occluder médical constitué d'un matériau à mémoire de forme configuré pour être inséré dans la cavité de l'appendice auriculaire gauche d'un patient, ledit occluder comprenant un lobe (1) et un disque (2) , le disque (2) est relié au lobe (1), le lobe est configuré pour se déplacer entre une première configuration à taille restreinte et au moins une deuxième configuration agrandie, la configuration restreinte a un diamètre de 1 à 40 % inférieur à la configuration élargie, le lobe comprend au moins un élément de restriction s'étendant à l'intérieur du lobe pour contrôler le diamètre du lobe, **caracterisé en ce que** laquelle élément de restriction est au moins une paire de fils chirurgicaux (4,5) s'étendant à l'intérieur du lobe dans la direction transversale et se croisant au milieu du lobe, chaque fil (4,5) est fixé à des parties opposées du lobe.

2. Occluder selon la revendication 1, dans lequel le diamètre de l'occluder dans la configuration restreinte est de 5 % à 30 % ou de 5 % à 20 % inférieur au diamètre de l'occluder dans la configuration agrandie.

3. Occluder selon la revendication 1, dans lequel l'occluder est une tresse en nitinol.

4. Occluder selon la revendication 1, dans lequel le disque est circulaire, elliptique ou en forme de poire.

5. Occluder selon la revendication 1, comprenant en outre au moins un élément d'ancrage.

6. Occluder selon la revendication 1, comprenant en outre un stylet avec un couteau.
